# FASCICULE DE BREVET EUROPEEN

(11) **EP 2 344 018 B1**
(45) Date de publication et mention de la délivrance du brevet: **01.08.2018**
(21) Numéro de dépôt: 09741347.0
(22) Date de dépôt: 01.09.2009
(51) Int. Cl.: A61B 1/00, A61B 1/018

(54) **INSTRUMENT POUR ENDOSCOPE**
INSTRUMENT FÜR EIN ENDOSKOP
INSTRUMENT FOR AN ENDOSCOPE

(30) Priorité: 01.09.2008 FR 0855854
(43) Date de publication de la demande: 20.07.2011
(73) Titulaire: Axess Vision Technology, 37000 Tours (FR)
(72) Inventeur: FRUCTUS, Olivier, 37530 Nazelles Negron (FR); MATHIEU, Nicolas, 69130 Ecully (FR)
(74) Mandataire: Thibault, Jean-Marc
(86) Numéro de dépôt international: PCT/FR2009/051649
(87) Numéro de publication internationale: WO 2010/023416

(56) Documents cités:
- WO-A2-2005/016181
- JP-A- 2003 339 624
- US-A- 5 025 778
- US-A- 5 257 617
- US-A- 5 503 616
- US-A- 5 876 329

## Description

La présente invention concerne le domaine technique de l'instrumentation relevant de tous les secteurs d'activités, permettant d'accéder, d'éclairer et d'examiner l'intérieur d'un corps au sens général tel une cavité ou un canal.

La présente invention concerne plus précisément mais non exclusivement un instrument pour un endoscope médical à usage unique ou réutilisable permettant d'éclairer et d'examiner la surface interne d'un organe creux, d'une cavité ou d'un conduit naturel ou artificiel du corps humain à des fins thérapeutiques, chirurgicales ou de diagnostic.

L'instrument selon l'invention est utilisé à des fins de diagnostic ou de chirurgie pour l'inspection de toutes parties internes du corps humain accessibles par les voies naturelles ou artificielles. Par exemple, l'instrument pour endoscope selon l'invention peut être utilisé dans le domaine des voies urinaires, des voies gastro-intestinales, du système respiratoire, du système cardio-vasculaire, de la trachée, de la cavité du sinus, du système de reproduction de la femme, de la cavité abdominale ou de tout autre partie du corps humain à explorer par une voie naturelle ou artificielle.

Dans le domaine technique ci-dessus, il existe différents types d'endoscope qui sont adaptés aux organes accessibles à observer. D'une manière générale, un endoscope comporte un tube d'insertion plus ou moins souple possédant une partie proximale de raccordement à un support d'actionnement permettant d'orienter le tube à l'intérieur de la voie d'insertion. Cet endoscope comporte également un système de visualisation généralement optique permettant d'éclairer et d'examiner à partir de la partie distale du tube d'insertion, l'organe, la cavité ou le conduit du corps humain. Dans de nombreuses applications, il apparaît avantageux d'amener au niveau de la partie distale du tube d'insertion, en association avec la zone d'accès, d'éclairage et d'observation, un ou plusieurs appareillages adaptés pour permettre de réaliser différentes fonctions telles que, l'amenée de fluide, l'amenée d'instruments, faire des prélèvements ou des opérations de chirurgie. La partie distale du tube d'insertion présente ainsi une section de sortie occupée d'une part, par la zone d'éclairage et d'observation et d'autre part, par l'orifice de passage pour un ou plusieurs appareillages.

Il doit être considéré que la section de sortie de la partie distale du tube d'insertion présente une valeur qui est limitée par la plus petite largeur de la voie d'accès naturelle ou artificielle dans laquelle le tube d'insertion est engagé.

Même si la miniaturisation des appareillages est en constante progression, le développement d'appareillages de plus en plus complexes impose de disposer d'une section de passage relativement importante au niveau de la partie distale du tube d'insertion. De façon analogue, la nécessité d'obtenir une bonne qualité d'image impose de disposer, au niveau de la partie distale du tube d'insertion, d'une zone d'éclairage et d'observation de surface suffisante.

Pour tenter de remédier à ces contraintes opposées, la demande de brevet internationale WO 2005/104 927 propose un endoscope comportant un tube d'insertion dont la partie distale est équipée de moyens d'articulation permettant le déploiement radial d'outillages et/ou du système optique d'observation. Il doit être noté que cette solution technique peut être mise en oeuvre uniquement dans le cadre d'un organe creux de section supérieure à la section de la voie d'accès pour l'endoscope. Il est à considérer que le fonctionnement des moyens d'articulation nécessite de disposer d'une zone de déploiement relativement importante, ce qui limite l'utilisation de cet endoscope. De plus, le déploiement radial des moyens d'articulation est à même d'endommager les tissus de l'organe inspecté. Par ailleurs, le déplacement des moyens d'articulation s'avère aléatoire et délicat à contrôler. Enfin, la décontamination de la partie distale du tube d'insertion s'avère difficile à mener à bien.

Dans le même but, le brevet US 5 025 778 décrit un endoscope comportant un tube d'insertion à l'extérieur duquel et sur toute sa longueur est emmanchée une chemise déformable aménagée pour délimiter, dans une position d'expansion, au moins un canal de passage. Avant l'insertion de l'endoscope dans le patient, le canal n'est pas formé de sorte que la chemise déformable est plaquée contre le tube d'insertion. Après l'insertion de l'endoscope dans le patient, un fluide ou un élément tubulaire est amené dans le canal de passage afin d'obtenir sa position d'expansion.

De manière similaire, le brevet US 5 503 616 décrit un endoscope comportant un tube d'insertion à l'extérieur duquel et sur toute sa longueur est rapportée une membrane déformable aménagée pour délimiter, dans une position d'expansion, un canal de passage pour un appareillage.

Ces solutions techniques entraînent une augmentation de la section de passage de l'endoscope en raison de l'ajout sur le tube d'insertion, d'une chemise ou d'une membrane déformable, même lorsque cette dernière n'est pas configurée en position d'expansion. Cette surépaisseur rapportée sur le tube d'insertion impose, pour une même section de passage de la voie d'accès, de réduire la taille du tube d'insertion et par suite de limiter ou réduire, la taille des appareillages et/ou du système de visualisation.

Par ailleurs, ces solutions techniques amènent à une augmentation de la section de passage de l'endoscope sur toute la longueur du tube d'insertion, ce qui est parfois impossible ou pour le moins, un risque de gêne pour le patient ou d'endommagement des tissus de la voie d'accès.

La présente invention vise donc à remédier aux inconvénients énoncés ci-dessus en proposant un instrument pour un endoscope au sens général et relevant plus particulièrement du domaine médical, cet instrument présentant une partie distale miniaturisée offrant une bonne qualité d'observation et la possibilité d'amener au niveau de cette partie distale divers appareillages, tout en endommageant pas les tissus ou organes environnants.

Un autre objet de l'invention est de proposer un instrument pour un endoscope dont le tube d'insertion ne présente pas de surépaisseur et qui permet d'optimiser l'exploitation de l'espace disponible dans la voie d'accès du patient lors du passage de l'endoscope.

Pour atteindre un tel objectif, l'instrument pour endoscope, comporte un tube d'insertion d'axe longitudinal possédant une partie proximale de raccordement à un support d'actionnement et une partie distale présentant une section de sortie occupée notamment d'une part par une zone de visualisation, et d'autre part, par au moins un orifice de sortie d'un passage pour au moins un appareillage destiné à occuper une position rétractée à l'intérieur du tube et une position de travail pour laquelle l'appareillage occupe au moins une partie de la section de sortie. Selon l'invention, la partie distale du tube d'insertion comporte sur une partie de sa longueur, à partir de la section de sortie, au moins une paroi déformable radialement permettant d'augmenter la section de sortie de la partie distale du tube lors du passage de l'appareillage de sa position rétractée dans laquelle l'appareillage ne sollicite pas la paroi déformable, à sa position de travail dans laquelle la paroi déformable est sollicitée radialement, la section de sortie de la partie distale correspondant à la section droite transversale du tube d'insertion en position rétractée de l'appareillage.

Selon une caractéristique avantageuse de réalisation, la partie distale du tube d'insertion est pourvu d'un organe d'étanchéité obturant l'orifice de sortie du passage pour l'appareillage. Cet organe d'étanchéité permet de ne pas contaminer ou de maintenir stérile l'appareillage tout au long du cheminement du tube d'insertion.

Par exemple, l'organe d'étanchéité est un bouchon éjectable ou une membrane déchirable, via l'appareillage ou une pression de fluide.

Selon un autre exemple de réalisation, l'appareillage comporte au moins un conduit tubulaire monté à l'intérieur du tube d'insertion, de manière coulissante par rapport au tube d'insertion.

Cet appareillage comporte au moins un outillage monté ou non dans le conduit tubulaire.

Selon une autre variante de réalisation, la partie distale du conduit tubulaire est pourvue d'au moins une enveloppe d'étanchéité.

Par exemple, l'enveloppe d'étanchéité présente une résistance supérieure à la résistance de l'organe d'étanchéité portée par la partie distale du tube d'insertion.

Selon un autre exemple, l'enveloppe d'étanchéité du conduit tubulaire est une enveloppe déchirable par l'outillage logé à l'intérieur du conduit tubulaire.

Selon une caractéristique de réalisation, le tube d'insertion comporte dans sa partie distale, un système de guidage de l'appareillage afin d'assurer l'expansion radiale de la paroi déformable lors du déplacement de l'appareillage.

Selon un exemple de réalisation, la partie distale du tube d'insertion est noyée dans un matériau déformable délimitant intérieurement le passage pour l'appareillage.

Selon une variante préférée de réalisation, la partie distale du tube d'insertion comporte un système de visualisation.

Il est à noter que la partie distale ou tête de l'instrument est amovible ou non par rapport au tube d'insertion.

Par exemple, le système de visualisation est noyé dans le matériau déformable.

Un autre objet de l'invention est de proposer un endoscope pourvu d'un instrument conforme à l'invention.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue schématique d'un premier exemple de réalisation d'un instrument pour endoscope conforme à l'invention.
La **Figure 2** est une vue en coupe élévation partielle de la partie distale de l'instrument tel qu'illustré à la **Fig. 1****.**
Les **Figures 3** et **4** sont des vues partielles respectivement en perspective et en coupe élévation de l'instrument conforme à l'invention dans une position de travail.
Les **Figures 5** à **7** sont des vues partielles en perspectives de la partie distale de l'instrument conforme à l'invention dans différentes positions caractéristiques d'utilisation.

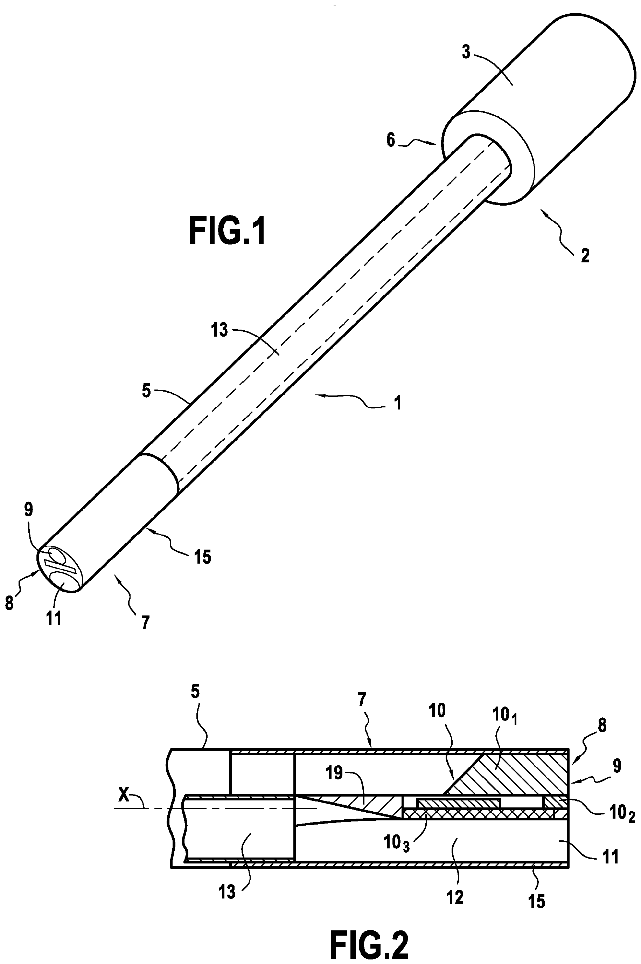

Tel que cela ressort plus précisément de la **Fig. 1****,** l'objet de l'invention concerne un instrument **1** pour un endoscope **2** adapté pour examiner l'intérieur d'un corps au sens général. De préférence, l'instrument **1** selon l'invention est particulièrement adapté pour être mis en oeuvre dans des applications médicales et équiper ainsi un endoscope médical permettant d'accéder et d'inspecter toute partie du corps humain, à partir d'une voie d'accès naturelle ou artificielle. Bien entendu, l'endoscope **2** permet d'éclairer et d'inspecter la surface interne d'un organe creux, d'une cavité ou d'un conduit du corps humain à des fins thérapeutiques, chirurgicales ou de diagnostic. Il doit être compris que l'instrument **1** est adapté pour équiper différents types d'endoscope qui sont adaptés aux organes accessibles par la voie naturelle dont le laryngoscope, le bronchoscope, l'oesophagoscope, le gastroscope, le duodénoscope, le cystoscope, l'hystéréoscope et le coloscope par exemple. Bien entendu, l'instrument **1** est adapté pour permettre d'accéder aussi par des voies pratiquées artificiellement dans le corps humain, à différentes parties internes du corps humain.

D'une manière classique, un endoscope médical **2** comporte un support ou un bloc d'actionnement **3** se présentant sous la forme généralement d'une poignée ou d'un bras robotisé, équipé de l'instrument **1** conforme à l'invention. L'instrument **1** est monté de manière solidaire ou de préférence, de manière séparable par rapport au support d'actionnement **3.** Dans ce dernier exemple préféré, l'endoscope **2** comporte entre l'instrument **1** et le bloc d'actionnement **3,** un système de connexion et de déconnexion adapté pour assurer rapidement une liaison au moins mécanique temporaire tout en offrant l'avantage de permettre une séparation facile de l'instrument **1** par rapport au support **3.** Le système de connexion et de déconnexion n'est pas décrit dans la mesure où il est bien connu de l'homme du métier et ne fait pas partie précisément de l'objet de l'invention.

Tel que cela ressort plus précisément des **Fig. 1** et **2****,** l'instrument **1** comporte un tube d'insertion **5** s'étendant selon un axe longitudinal **X** et de section droite transversale de préférence circulaire. Ce tube d'insertion **5** comporte une flexibilité plus ou moins importante et se trouve réalisé en un matériau biocompatible. Le tube d'insertion **5** possède une partie proximale **6** de raccordement au support d'actionnement **3** et une partie distale **7** opposée formant la tête de l'instrument **1.** De manière avantageuse, l'endoscope **1** comporte un dispositif permettant le pliage ou le béquillage de la partie distale **7** de l'instrument **1.** Le dispositif d'actionnement non représenté mais réalisé de toute manière appropriée comporte généralement un ou plusieurs câbles d'actionnement fixés à la partie distale **7** de l'instrument et raccordés à un système d'actionnement monté dans le support **3.**

Tel que cela ressort plus précisément de la **Fig. 2****,** la partie distale **7** de l'instrument **1** présente à son extrémité libre, une face transversale **8** qui délimite la section de sortie de la tête **7.** Cette section de sortie **8** est occupée notamment d'une part, par une zone de visualisation **9** et d'autre part, par au moins un et dans l'exemple illustré, un orifice **11** d'un passage **12** pour au moins un appareillage **13.** Dans l'exemple illustré par les dessins, la face transversale **8** s'étend sensiblement perpendiculairement à l'axe longitudinal **X** du tube **5.** Bien entendu, la face transversale **8** peut s'étendre de manière plus ou moins inclinée par rapport à l'axe longitudinal **X.**

La zone de visualisation **9** correspond à une partie de la face transversale **8** de la partie distale **7** à partir de laquelle un système de visualisation **10** permet d'observer ou d'inspecter une partie interne du corps humain. De manière avantageuse, le système de visualisation **10** est un système d'imagerie. Par exemple, le système de visualisation **10** comporte un système **10₁** de formation d'une image monté à l'intérieur de la partie distale **7** et relié à un faisceau de transmission s'étendant à l'intérieur du tube **5** et communiquant à une unité d'acquisition et de traitement d'images. Par exemple, le système de formation d'images **10₁** comporte une ou plusieurs lentilles optiques reliées à tous types de transmission d'images. De préférence, le système optique d'observation **10** comporte un éclairage **10₂** comportant une source lumineuse telle une diode électroluminescente fixée sur un circuit imprimé **10₃.** Bien entendu, l'éclairage **10₂** est relié à un faisceau de connexion électrique s'étendant à l'intérieur du tube **5** pour être relié en amont de la partie proximale **6** de l'instrument, à une source électrique. Dans l'exemple de réalisation décrit ci-dessus, le système de visualisation **10** est de nature optique et associé de préférence à un éclairage mais il est clair que le système de visualisation **10** peut être réalisé différemment. Par exemple, le système de visualisation **10** peut mettre en oeuvre un système ionisant ou de rayonnement tel que par ultrasons.

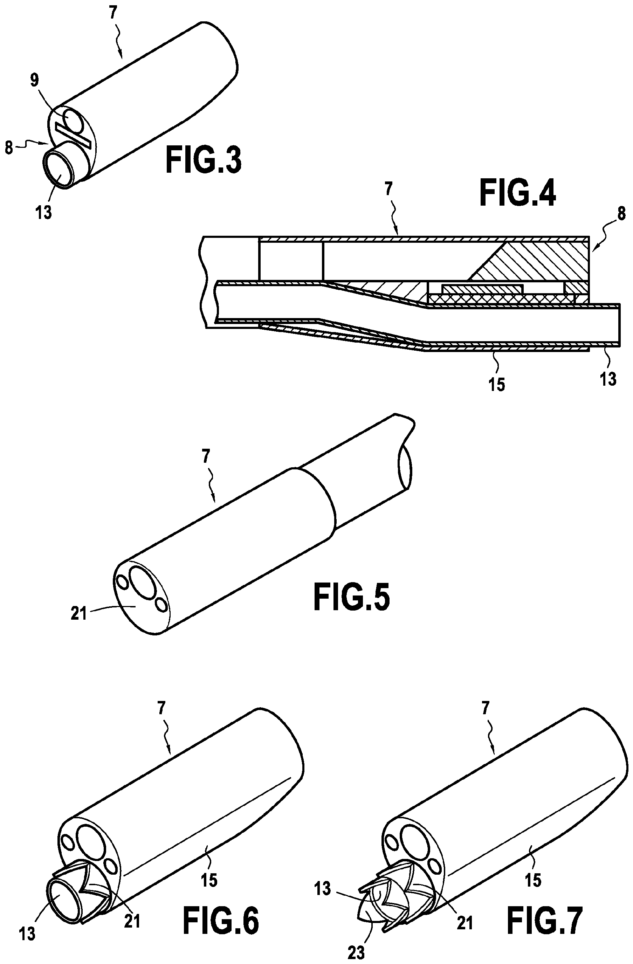

L'appareillage **13** est destiné à occuper une position rétractée à l'intérieur du tube d'insertion **5** comme illustré aux **Fig. 1** et **2****,** et une position de travail telle qu'illustrée aux **Fig. 3** et **4****,** pour laquelle l'appareillage **13** occupe au moins une partie de la section de sortie **8.** D'une manière générale, l'appareillage **13** équipant l'instrument **1** dépend de la nature de la ou des opérations à réaliser par l'endoscope. L'appareillage **13** correspond, de manière non limitative, à un ou plusieurs équipements, outils, capteurs, matériels ou accessoires. Cet appareillage **13** peut relever de divers domaines et être de nature par exemple, mécanique, électrique, calorifique, magnétique, chimique, fluidique, solide, etc. en vue de réaliser diverses opérations telles que par exemple, des incisions, destructions, prélèvements, mesures, apport d'un matériau (gaz, liquide, solide).

Dans l'exemple de réalisation illustré, l'appareillage **13** comporte un conduit tubulaire monté à l'intérieur du tube d'insertion **5,** de manière coulissante par rapport au tube **5.** Ce conduit tubulaire **13** est relié à tous moyens appropriés permettant d'assurer le coulissement relatif du conduit tubulaire à l'intérieur du tube d'insertion **5.** Ce conduit tubulaire **13** permet ainsi d'amener à l'extrémité du tube d'insertion **5** tous types d'équipements, d'outillages ou de matériels de toutes natures. Ces équipements et autres peuvent être rapportés ou montés à l'intérieur du tube d'insertion **5.** Par exemple, dans le cas de l'apport d'un fluide à la partie distale de l'instrument, le conduit tubulaire **13** peut soit être raccordé à une source d'amenée de fluide au niveau de la partie proximale de l'instrument et/ou soit incorporer une quantité de fluide et/ou d'un matériau qui est destiné à être délivré à l'extrémité distale de l'instrument **1.**

Bien entendu, l'appareillage **13** peut comporter un ou plusieurs tubes dans lesquels les équipements et autres sont montés. Par ailleurs, l'appareillage **13** peut comporter les équipements et autres montés directement à l'intérieur du tube d'insertion **5,** c'est-à-dire que l'appareillage **13** ne comporte pas de conduit tubulaire. Dans le cas où l'appareillage **13** comporte plusieurs équipements et autres, ces derniers peuvent sortir de la partie distale **7** par le même orifice **11** ou par plusieurs orifices **11** de passages **12** communiquant avec l'intérieur du tube d'insertion **5.**

Conformément à l'invention, la partie distale **7** du tube d'insertion **5** comporte sur une partie de sa longueur, à partir de la section de sortie **8,** au moins une paroi déformable radialement **15** permettant d'augmenter la section de sortie **8** de la partie distale du tube lors du passage d'au moins un appareillage **13** de sa position rétractée à sa position de travail. Cette paroi déformable **15** est réalisée de toute manière appropriée pour permettre une extension radiale de la partie distale **7** du tube **5** lors de l'amenée de l'appareillage **13** jusqu'à la section de sortie **8.**

Bien entendu, la section droite transversale de l'appareillage **13** est avantageusement supérieure à la section droite transversale de l'orifice de sortie **11** au repos ou en position rétractée de l'appareillage **13.** La position rétractée de l'appareillage **13** est choisie en amont de la section de sortie **8** à un endroit n'affectant pas la section droite transversale du tube d'insertion **5.** Dans l'exemple illustré à la **Fig. 2****,** la position rétractée de l'appareillage **13** est schématisée à l'entrée de la partie distale **7** mais il est clair que cette position rétractée peut correspondre à toute position à l'intérieur de l'instrument **1** ou du support **3.**

Dans l'exemple de réalisation illustré, la paroi déformable **15** est réalisée par une partie du matériau déformable constituant la partie distale **7** du tube d'insertion. Selon cet exemple de réalisation, la partie distale **7** se présente sous la forme d'un bloc ou manchon dans lequel le système de visualisation **10** est noyé. Selon cet exemple, le bloc de matériau délimite intérieurement un alésage pour former le passage **12** qui communique avec l'intérieur du tube **5** et s'ouvre à l'extrémité par l'orifice de sortie **11.**

Selon cet exemple de réalisation, la partie distale **7** se présente donc sous la forme d'un bloc en un matériau souple dans lequel le dispositif de visualisation **10** est noyé et permettant de délimiter le passage **12** pour l'appareillage. Cette partie distale **7** est raccordée par tous moyens appropriés au tube d'insertion **5.** Cette partie distale ou tête **7** est raccordée au tube d'insertion **5** à l'aide de moyens d'assemblage temporaire ou définitif. Ainsi, la tête ou partie distale **7** peut être amovible ou non par rapport au tube d'insertion **5.** Cette tête ou partie distale **7** est donc montée dans le prolongement du tube d'insertion **5** en présentant, en position rétractée de l'appareillage **13,** une section droite transversale égale à la section droite transversale du tube d'insertion **5.**

Bien entendu, la paroi déformable radialement **10** peut être réalisée de manière différente. Cette paroi déformable **10** peut être réalisée par exemple par une membrane souple fixée sur un corps rigide. Dans ce cas, la partie distale **7** constitue un corps essentiellement rigide équipé de la paroi déformable **10.** Cette paroi déformable **10** peut présenter une déformation qui est définitive ou temporaire lorsque l'appareillage **13** retourne à sa position rétractée à l'intérieur du tube **5.** Par ailleurs, la déformation peut provenir non pas d'un effort mécanique comme expliqué ci-dessus mais par exemple d'un apport calorifique, ionisant ou autre.

Selon une caractéristique avantageuse, la partie distale **7** du tube d'insertion **5** comporte un système **19** de guidage de l'appareillage **13** afin d'assurer l'expansion radiale de la paroi déformable **15** lors du déplacement de l'appareillage. Tel que cela ressort plus précisément des **Fig. 2** et **4****,** le système de guidage **19** se présente sous la forme d'une rampe ou d'un cône débouchant dans l'alésage **12** permettant de guider progressivement l'appareillage **13** afin qu'il passe de sa position rétractée à sa position de travail.

Il ressort de la description qui précède, qu'en position rétractée de l'appareillage **13,** la section de sortie **8** de la partie distale **7** de l'instrument **1** correspond à la section droite transversale du tube d'insertion **5** (**Fig. 1, 2**). En d'autres termes, la partie distale **7** de l'instrument **1** présente, en position rétractée de l'appareillage **13,** une section droite transversale qui est égale à la section droite transversale du tube d'insertion **5,** cette partie distale **7** de l'instrument **1** ne comportant pas de surépaisseur à l'extérieur du tube d'insertion **5.** Ainsi, en position rétractée de l'appareillage **13,** le tube d'insertion **5** présente une section droite transversale constante sur toute la longueur de sa partie proximale **6** à sa partie distale **7.**

Lorsque la partie distale **7** occupe une position dans laquelle un appareillage **13** doit être amené au niveau de sa section de sortie **8,** le déplacement ou le coulissement de l'appareillage **13** par rapport au tube **5,** conduit à une déformation radiale de la paroi déformable **15** de sorte qu'au niveau de la section de sortie **8,** la section de l'orifice de sortie **11** augmente (**Fig. 3, 4**). En d'autres termes, en position de travail, la section de sortie **8** de la partie distale **7** du tube d'insertion **5** est supérieure à la section de sortie **8** de la partie distale **7** du tube d'insertion **5,** en position rétractée de l'appareillage **13.** Le tube d'insertion **5** possède donc une section de sortie **8** qui augmente lorsque l'appareillage **13** passe d'une position rétractée à une position de travail. Il est à noter que la section droite transversale du tube d'insertion **5** est augmentée sur une longueur axiale limitée à au plus la partie distale **7** du tube d'insertion **5.** Typiquement, la partie distale **7** du tube d'insertion **5** qui présente une variation de section droite transversale due à l'appareillage correspond à une longueur axiale inférieure ou égale à deux fois le diamètre du tube d'insertion **5.**

L'accroissement du diamètre de la partie distale **7** autorise l'amenée de divers appareillages **13** tout en évitant d'endommager les tissus environnants. Il apparaît ainsi possible de disposer à la partie distale de l'instrument **1,** d'un appareillage **13** présentant un encombrement ou une section de sortie, bien plus importante que la section de sortie de l'orifice **11** au repos.

Selon une caractéristique avantageuse de l'objet de l'invention illustrée plus précisément aux **Fig. 5** et **6****,** la partie distale **7** du tube d'insertion **5** est pourvue d'un organe d'étanchéité **21** obturant l'orifice de sortie **11** du passage pour l'appareillage. Cet organe d'étanchéité **21** est un bouchon éjectable, une membrane déchirable, ou autres, via l'appareillage **13** ou une pression de fluide. Cet organe d'étanchéité **21** obture ainsi l'orifice de sortie **11.** Tel que cela ressort plus précisément de la **Fig. 6****,** le coulissement de l'appareillage **13** pour assurer sa sortie au niveau de l'extrémité de la partie distale **7** conduit à la déformation ou au déchirage de la membrane d'étanchéité **21** (ou à l'éjection du bouchon d'étanchéité). Bien entendu, l'amenée d'un fluide sous pression au niveau de l'orifice de sortie **11** peut assurer également la suppression de l'organe d'étanchéité **21.**

Selon une autre caractéristique avantageuse de réalisation illustrée à la **Fig. 7****,** la partie distale **7** du conduit tubulaire **13** est pourvue d'au moins une enveloppe d'étanchéité **23.** Cette enveloppe d'étanchéité **23** est déchirable par l'outillage **13** logé à l'intérieur du conduit tubulaire **13.** Avantageusement, cette enveloppe d'étanchéité **23** présente une résistance supérieure à la résistance de l'organe d'étanchéité **21** porté par la partie distale **7.**

L'invention n'est pas limitée aux exemples décrits et représentés car diverses modifications peuvent y être apportées sans sortir de son cadre.

## Revendications

1. Instrument pour endoscope, comportant un tube d'insertion (**5**) d'axe longitudinal (**X**) de section droite transversale par rapport à cet axe longitudinal et possédant une partie proximale (**6**) de raccordement à un support d'actionnement et une partie distale (**7**) présentant une section de sortie (**8**) occupée notamment d'une part par une zone de visualisation (**9**), et d'autre part, par au moins un orifice de sortie (**11**) d'un passage (**12**) pour au moins un appareillage (**13**) destiné à occuper une position rétractée à l'intérieur du tube (**5**) et une position de travail pour laquelle l'appareillage occupe au moins une partie de la section de sortie (**8**), la partie distale (**7**) du tube d'insertion comportant sur une partie de sa longueur, à partir de la section de sortie (**8**), au moins une paroi (**15**) déformable radialement permettant d'augmenter la section de sortie de la partie distale (**7**) du tube (**5**) lors du passage de l'appareillage (**13**) de sa position rétractée dans laquelle l'appareillage ne sollicite pas la paroi déformable, à sa position de travail dans laquelle la paroi déformable (15) est sollicitée radialement, la section de sortie (**8**) de la partie distale (**7**) correspondant à la section droite transversale du tube d'insertion (**5**), en position rétractée de l'appareillage (**13**), **caractérisé en ce que** le tube d'insertion (**5**) comporte dans sa partie distale (**7**), un système de guidage (**19**) de l'appareillage (**13**) se présentant sous la forme d'une rampe ou d'un cône débouchant dans un alésage qui forme le passage (**12**) pour guider progressivement l'appareillage (**13**) afin qu'il passe de sa position rétractée à sa position de travail en entraînant l'expansion radiale de la paroi déformable (15) lors du déplacement de l'appareillage.

2. Instrument médical selon la revendication 1, **caractérisé en ce que** la partie distale (**7**) du tube d'insertion est pourvu d'un organe d'étanchéité (**21**) obturant l'orifice de sortie (**11**) du passage pour l'appareillage (**13**).

3. Instrument selon la revendication 2, **caractérisé en ce que** l'organe d'étanchéité (**21**) est un bouchon éjectable ou une membrane déchirable, via l'appareillage ou une pression de fluide.

4. Instrument selon l'une des revendications 1 à 3, **caractérisé en ce que** l'appareillage (**13**) comporte au moins un conduit tubulaire monté à l'intérieur du tube d'insertion, de manière coulissante par rapport au tube d'insertion.

5. Instrument selon l'une des revendications 1 à 4, **caractérisé en ce que** l'appareillage (**13**) comporte au moins un outillage monté ou non dans le conduit tubulaire.

6. Instrument selon la revendication 4 ou 5, **caractérisé en ce que** la partie distale (**7**) du conduit tubulaire (**13**) est pourvue d'au moins une enveloppe d'étanchéité (**23**).

7. Instrument selon la revendication 6, **caractérisé en ce que** l'enveloppe d'étanchéité (**23**) présente une résistance supérieure à la résistance de l'organe d'étanchéité (**21**) portée par la partie distale (**7**) du tube d'insertion.

8. Instrument selon la revendication 5, **caractérisé en ce que** l'enveloppe d'étanchéité (**23**) du conduit tubulaire (**13**) est une enveloppe déchirable par l'outillage logé à l'intérieur du conduit tubulaire (**13**).

9. Instrument selon l'une des revendications 1 à 8, **caractérisé en ce que** la partie distale (**7**) du tube d'insertion (**5**) est noyée dans un matériau déformable délimitant intérieurement le passage (**12**) pour l'appareillage.

10. Instrument selon la revendication 1, **caractérisé en ce que** la partie distale (**7**) du tube d'insertion (**5**) comporte un système de visualisation (**10**).

11. Instrument selon les revendications 9 et 10, **caractérisé en ce que** le système de visualisation (**10**) est noyé dans le matériau déformable.

12. Instrument selon l'une des revendications 1 à 11, **caractérisé en ce que** la partie distale (**7**) est amovible ou non par rapport au tube d'insertion (**5**).

13. Endoscope **caractérisé en ce qu'**il comporte au moins un instrument (**1**) conforme à l'une des revendications 1 à 12.

## Patentansprüche

1. Instrument für ein Endoskop, umfassend ein Einführrohr (5) mit einer Längsachse (X), mit einem Querschnitt in Bezug auf diese Längsachse, und mit einem proximalen Teil (6) zur Verbindung mit einem Betätigungshalter und einem distalen Teil (7), der einen Austrittsquerschnitt (8) aufweist, welcher insbesondere einerseits durch einen Anzeigebereich (9) und andererseits durch wenigstens eine Austrittsöffnung (11) eines Durchgangs (12) für wenigstens eine Einrichtung (13) eingenommen ist, die dazu bestimmt ist, eine eingezogene Position innerhalb des Rohres (5) und eine Arbeitsposition einzunehmen, für die die Einrichtung wenigstens einen Teil des Austrittsquerschnitts (8) einnimmt, wobei der distale Teil (7) des Einführrohres auf einem Teil seiner Länge, von dem Austrittsquerschnitt (8) ausgehend, wenigstens eine radial verformbare Wand (15) umfasst, die ermöglicht, den Austrittsquerschnitt des distalen Teils (7) des Rohres (5) beim Übergang der Einrichtung (13) von ihrer eingezogenen Position, in der die Einrichtung die verformbare Wand nicht belastet, in ihre Arbeitsposition, in der die verformbare Wand (15) radial belastet ist, zu vergrößern, wobei der Austrittsquerschnitt (8) des distalen Teils (7) dem Querschnitt des Einführrohres (5) in der eingezogenen Position der Einrichtung (13) entspricht, **dadurch gekennzeichnet, dass** das Einführrohr (5) in seinem distalen Teil (7) ein System zum Führen (19) der Einrichtung (13) umfasst, das in Form einer Rampe oder eines Konus vorliegt, welche(r) in eine Bohrung mündet, die den Durchgang (12) bildet, um die Einrichtung (13) schrittweise zu führen, damit sie unter Bewirken der radialen Ausdehnung der verformbaren Wand (15) während der Bewegung der Einrichtung von ihrer eingezogenen Position in ihre Arbeitsposition übergeht.

2. Medizinisches Instrument nach Anspruch 1, **dadurch gekennzeichnet, dass** der distale Teil (7) des Einführrohres mit einem Dichtungselement (21) versehen ist, das die Austrittsöffnung (11) des Durchgangs für die Einrichtung (13) verschließt.

3. Instrument nach Anspruch 2, **dadurch gekennzeichnet, dass** das Dichtungselement (21) ein(e) über die Einrichtung oder einen Fluiddruck auswerfbarer Stopfen oder zerreißbare Membran ist.

4. Instrument nach einem der Ansprüche 1 bis 3, **dadurch gekennzeichnet, dass** die Einrichtung (13) wenigstens eine rohrförmige Leitung umfasst, die innerhalb des Einführrohres, gegenüber dem Einführrohr verschieblich, angebracht ist.

5. Instrument nach einem der Ansprüche 1 bis 4, **dadurch gekennzeichnet, dass** die Einrichtung (13) wenigstens ein Werkzeug aufweist, das in der rohrförmigen Leitung angebracht ist oder nicht.

6. Instrument nach Anspruch 4 oder 5, **dadurch gekennzeichnet, dass** der distale Teil (7) der rohrförmigen Leitung (13) mit wenigstens einer Dichtungshülle (23) versehen ist.

7. Instrument nach Anspruch 6, **dadurch gekennzeichnet, dass** die Dichtungshülle (23) einen Widerstand aufweist, der größer ist als der Widerstand des Dichtungselements (21), das von dem distalen Teil (7) des Einführrohres getragen wird.

8. Instrument nach Anspruch 5, **dadurch gekennzeichnet, dass** die Dichtungshülle (23) der rohrförmigen Leitung (13) eine Hülle ist, welche durch das innerhalb der rohrförmigen Leitung (13) aufgenommene Werkzeug zerreißbar ist.

9. Instrument nach einem der Ansprüche 1 bis 8, **dadurch gekennzeichnet, dass** der distale Teil (7) des Einführrohres (5) in ein verformbares Material eingebettet ist, das den Durchgang (12) für die Einrichtung innen begrenzt.

10. Instrument nach Anspruch 1, **dadurch gekennzeichnet, dass** der distale Teil (7) des Einführrohres (5) ein Anzeigesystem (10) umfasst.

11. Instrument nach den Ansprüchen 9 und 10, **dadurch gekennzeichnet, dass** das Anzeigesystem (10) in das verformbare Material eingebettet ist.

12. Instrument nach einem der Ansprüche 1 bis 11, **dadurch gekennzeichnet, dass** der distale Teil (7) gegenüber dem Einführrohr (5) lösbar ist oder nicht.

13. Endoskop, **dadurch gekennzeichnet, dass** es wenigstens ein Instrument (1) gemäß einem der Ansprüche 1 bis 12 umfasst.

## Claims

1. An endoscope instrument comprising an insertion tube (**5**) of longitudinal axis (**X**) with a straight transverse section relative to this longitudinal axis and possessing a proximal portion (**6**) for connection to an actuation support and a distal portion (**7**) presenting an outlet section (**8**) occupied in particular firstly by a viewing zone (**9**) and secondly by at least one outlet orifice (**11**) of a passage (**12**) for at least one appliance (**13**) for occupying a retracted position inside the tube (**5**) and a working position in which the appliance occupies at least a portion of the outlet section (**8**), the distal portion (**7**) of the insertion tube including at least one radially deformable wall (**15**) over a portion of its length extending from the outlet section (**8**), thereby enabling the outlet section of the distal portion (**7**) of the tube (**5**) to be increased on the appliance (**13**) passing from its retracted position in which the appliance does not stress the deformable wall to its working position in which the deformable wall (**15**) is stressed radially, the outlet section (**8**) of the distal portion (**7**) corresponding to the right cross-section of the insertion tube (**5**) in the retracted position of the appliance (**13**), **characterized in that** the insertion tube (**5**) includes, in its distal portion (**7**), a system (**19**) for guiding the appliance (**13**) assuming the form of a ramp or a cone emerging in a bore that forms the passage (**1**2) for gradually guiding the appliance (**13**) so that it passes from its retracted position to its working position while causing the radial expansion of the deformable wall (**15**) during the movement of the appliance.

2. The medical instrument according to claim 1, **characterized in that** the distal portion (**7**) of the insertion tube is provided with a sealing member (**21**) closing the outlet orifice (**11**) of the passage for the appliance (**13**).

3. The instrument according to claim 2, **characterized in that** the sealing member (**21**) is a plug that is ejectable or a membrane that is tearable by the appliance or by fluid pressure.

4. The instrument according to one of claims 1 to 3, **characterized in that** the appliance (**13**) comprises at least one tubular duct mounted inside the insertion tube so as to be slidable relative to the insertion tube.

5. The instrument according to one of claims 1 to 4, **characterized in that** the appliance (**13**) includes at least one tool optionally mounted in the tubular duct.

6. The instrument according to claim 4 or claim 5, **characterized in that** the distal portion (**7**) of the tubular duct (**13**) is provided with at least one sealing envelope (**23**).

7. The instrument according to claim 6, **characterized in that** the sealing envelope (**23**) presents mechanical strength that is greater than that of the sealing member (**21**) carried by the distal portion (**7**) of the insertion tube.

8. The instrument according to claim 5, **characterized in that** the sealing envelope (**23**) of the tubular duct (**13**) is an envelope that is tearable by the tooling housing inside the tubular duct (**13**).

9. The instrument according to one of claims 1 to 8, **characterized in that** the distal portion (**7**) of the insertion tube (**5**) is embedded in a deformable material internally defining the passage (**12**) for the appliance.

10. The instrument according to claim 1, **characterized in that** the distal portion (**7**) of the insertion tube (**5**) includes a viewing system (**10**).

11. The instrument according to claims 9 and 10, **characterized in that** the viewing system (**10**) is embedded in the deformable material.

12. The instrument according to one of claims 1 to 11, **characterized in that** the distal portion (**7**) is optionally removable relative to the insertion tube (**5**).

13. An endoscope, **characterized in that** it includes at least one instrument (**1**) in accordance with any one of claims 1 to 12.
